# EUROPEAN PATENT SPECIFICATION

(11) **EP 3 514 402 B1**
(45) Date of publication and mention of the grant of the patent: **02.08.2023**
(21) Application number: 16829396.7
(22) Date of filing: 14.09.2016
(51) Int. Cl.: F16F 3/04, A61M 25/09, B21F 15/04

(54) **CONNECTION STRUCTURE, AND GUIDE WIRE PROVIDED WITH CONNECTION STRUCTURE**
VERBINDUNGSSTRUKTUR UND FÜHRUNGSDRAHT MIT DER VERBINDUNGSSTRUKTUR
STRUCTURE DE RACCORDEMENT ET FIL-GUIDE POURVU D'UNE STRUCTURE DE RACCORDEMENT

(43) Date of publication of application: 24.07.2019
(73) Proprietor: Asahi Intecc Co., Ltd., Seto-shi, Aichi 489-0071 (JP)
(72) Inventor: YONEZAWA Satoshi, Nagoya-shi Aichi 463-0024 (JP)
(74) Representative: Winter, Brandl - Partnerschaft mbB
(86) International application number: PCT/JP2016/077176
(87) International publication number: WO 2018/051440

(56) References cited:
- JP-A- 2004 298 635
- JP-A- 2010 084 932
- JP-A- 2011 251 046
- JP-A- 2012 254 233
- JP-U- H0 623 543
- US-A- 2 756 783
- US-A1- 2008 051 676
- US-A1- 2014 046 302

## Description

### TECHNICAL FIELD

The present invention relates to a connection structure for connecting two metals and a guide wire having the connection structure.

### BACKGROUND ART

To date, various connection structures for connecting two metals, in particular a structure in which a connected portion is flexible have been proposed.

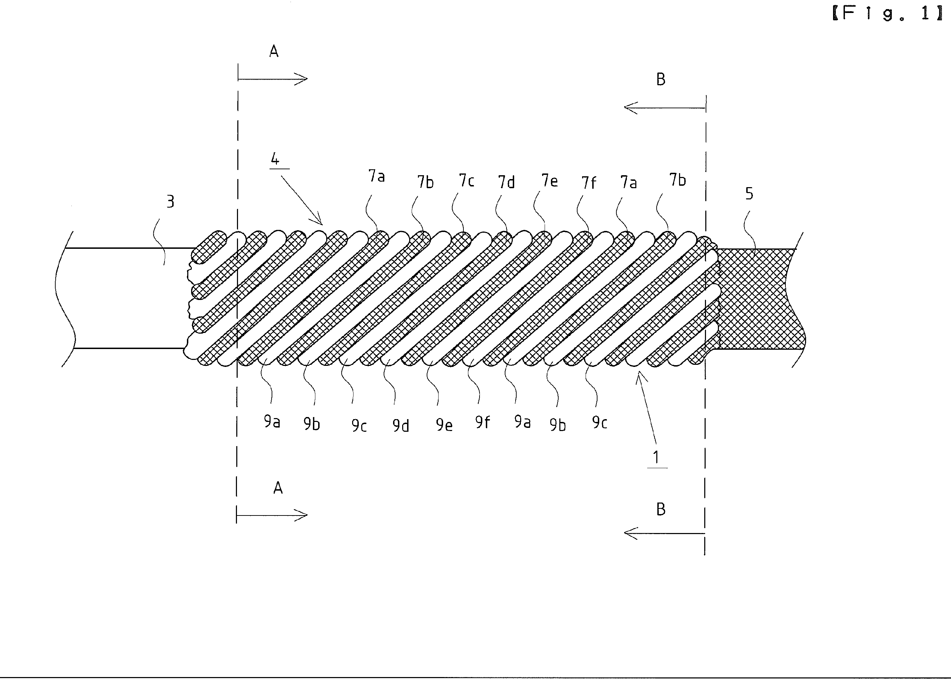

For example, JP 2000 145660 A describes a flexible shaft (a connection structure) in which a axial part 8 is connected to a coupling 10 through a bendable part 9 comprising a coiled spring, the coiled spring being a multi-thread coiled spring (see Fig. 1 and others).

According to the flexible shaft described in JP 2000 145660 A, the axial part is connected to the coupling through the coiled spring, and the coiled spring serves as a connected portion. This enables the connected portion to be flexible.

However, the flexible shaft described in JP 2000 145660 A has the following disadvantage: the axial part is connected to the coupling through the entire circumference of the coiled spring, and thus the flexible shaft may somewhat be difficult to be bent due to the repulsive force of the coiled spring when the axial part and the coupling are held and attempted to be bent.

Further, if the axial part and the coupling comprise dissimilar metals, this dissimilarity needs to be taken into account when a connected portion is formed.

Moreover, such a connection structure may potentially be used in a medical device, in particular a guide wire which is used inside a complicatedly winding blood vessel.

US 2008/051676 A1 discloses a connection structure comprising wire guide and a extension wire.

JP 2010 084932 A discloses a coil spring device comprising two independent coil springs.

US 2014/046302 A1 discloses a medical wire guide comprising a mandril having a distal tip, a first coil coupled with the distal tip of the mandril, and a second coil configured to releasably engage with the mandril or the first coil.

US 2 756 783 A discloses a connection structure comprising a coiled part and a coiled part. The coiled part and the coiled part are wound around each other.

### SUMMARY OF INVENTION

### Technical Problem

The present invention is made to solve the above problems. An object of the present invention is to provide a connection structure for connecting two metals in which the flexibility of a connected portion can be further improved, and in particular an appropriate connection can be provided even when the two metals are dissimilar.

### Solution to Problem

In order to achieve the above object, a first aspect of the present invention is characterized by a connection structure between a first metal rod and a second metal rod. The first metal rod comprises a first metal and the second metal rod comprises a second metal. A multi-thread coil formed by winding first element wires comprising the first metal, and second element wires comprising the second metal is arranged. The multi-thread coil bestrides the first metal rod and the second metal rod. The first metal rod is connected to the first element wires of the multi-thread coil, and the second metal rod is connected to the second element wires of the multi-thread coil. The first element wires and the second element wires are arranged around cross-sectional circumferences at an equal angle so as to cover the entire cross-sectional circumferences of the first metal rod and the second metal rod.

Further, a second aspect of the present invention is characterized by the connection structure according to the first aspect of the invention, in which the multi-thread coil comprises the first elemental wires and the second element wires adjacently wound one by one, and the first element wires are connected to the first metal rod so as to sandwich the second element wires, and the second element wires are connected to the second metal rod so as to sandwich the first element wires.

Moreover, a third aspect of the present invention is characterized by the connection structure according to the first or second aspect of the invention, in which the first metal comprises a stainless steel alloy, and the second metal comprises a nickel-titanium alloy.

Furthermore, a fourth aspect of the present invention is characterized by a guide wire comprising a core shaft having the connection structure according to any one of the first to third aspects of the invention, and a coil body covering the front end of the core shaft and connected to the first metal rod or the second metal rod.

### Advantageous Effects of Invention

In the connection structure between the first metal rod and the second metal rod according to the first aspect of the present invention, a multi-thread coil formed by winding first elemental wires comprising the first metal and second element wires comprising the second metal is arranged, the multi-thread coil bestriding the first metal rod and the second metal rod, and the first metal rod is connected to the first element wires of the multi-thread coil, and the second metal rod is connected to the second element wires of the multi-thread coil. This can improve the flexibility of a connected portion formed by the multi-thread coil.

Further, in the connection structure according to the second aspect, the multi-thread coil comprises the first elemental wires and the second element wires adjacently wound one by one, and the first element wires are connected to the first metal rod so as to sandwich the second element wires, and the second element wires are connected to the second metal rod so as to sandwich the first element wires in the connection structure according to the first aspect of the invention. This can prevent distortion of the shape of the multi-thread coil as much as possible, and can improve the flexibility of the connected portion.

Moreover, in the connection structure according to the third aspect, the first metal comprises a stainless steel alloy, and the second metal comprises a nickel-titanium alloy in the connection structure according to the first or second aspect of the invention. This can improve the flexibility of the connected portion, and can also provide an appropriate connection even when dissimilar metals difficult to be directly connected are used.

Furthermore, in the guide wire according to the fourth aspect, provided is a guide wire comprising a core shaft having the connection structure according to any one of the first to third aspects of the invention, and a coil body covering the front end of the core shaft and connected to the first metal rod or the second metal rod. This can improve the flexibility of the connected portion of the core shaft, allowing the guide wire itself to easily follow a winding blood vessel.

### BRIEF DESCRIPTION OF DRAWINGS

Fig. 1 shows a side view of a connection structure according to a first embodiment of the present invention.
Fig. 2 shows a cross-sectional side view of the connection structure according to the first embodiment.
Fig. 3 shows a cross-sectional view at A-A of Figure 1.
Fig. 4 shows a cross-sectional view at B-B of Figure 1.
Fig. 5 shows a side view of a connection structure according to a second embodiment.
Fig. 6 shows a cross-sectional side view of the connection structure according to the second embodiment.
Fig. 7 shows a schematic side view of a guide wire according to a third embodiment.
Fig. 8 shows a schematic cross-sectional side view of the guide wire according to the third embodiment.

### DESCRIPTION OF EMBODIMENTS

Below, the aforementioned embodiments of the present invention will be described with reference to the drawings.

### First embodiment

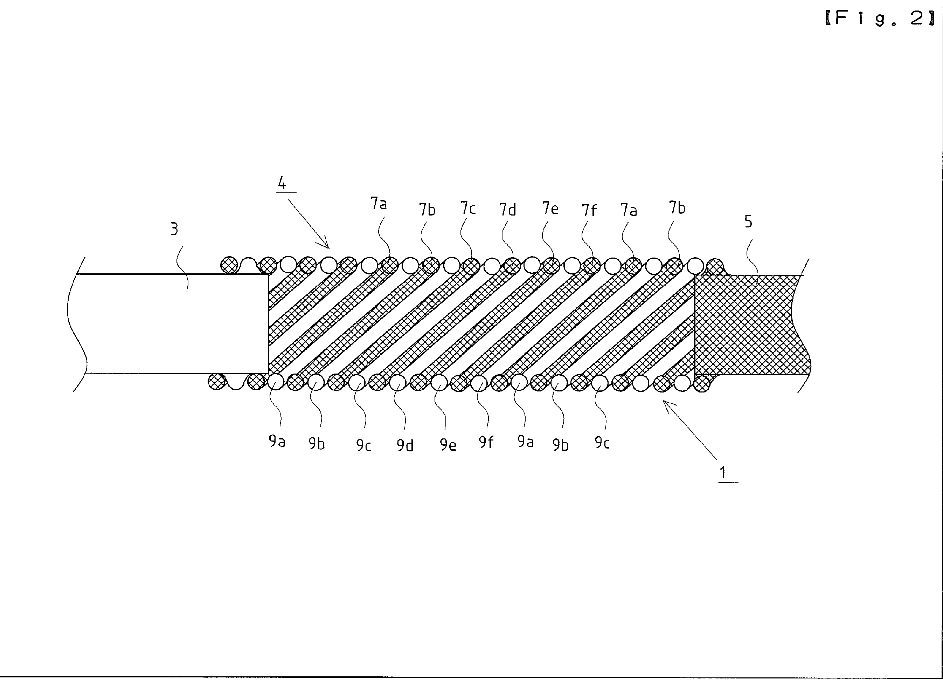

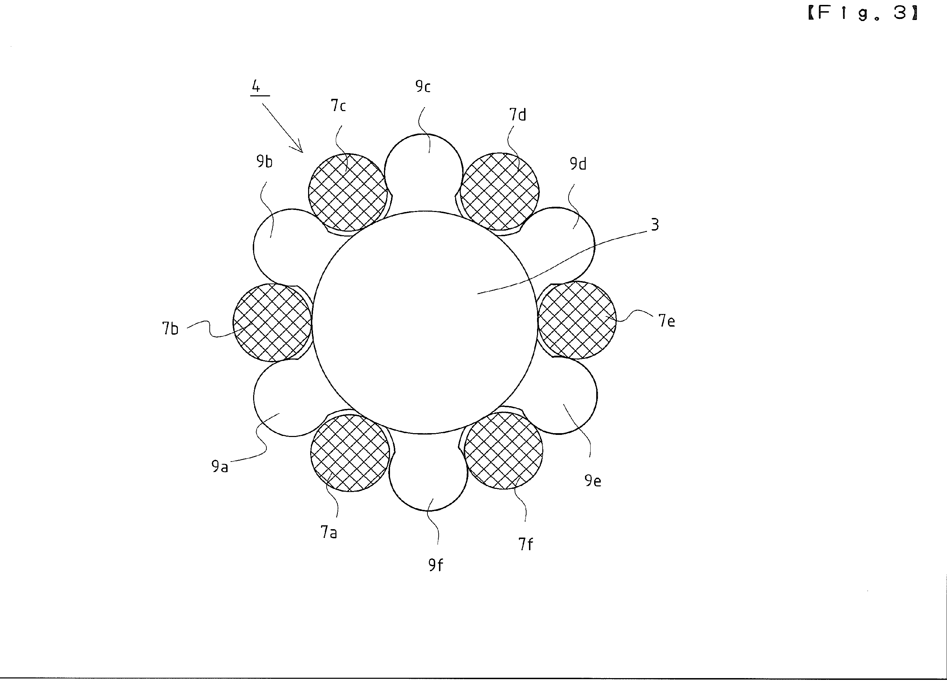

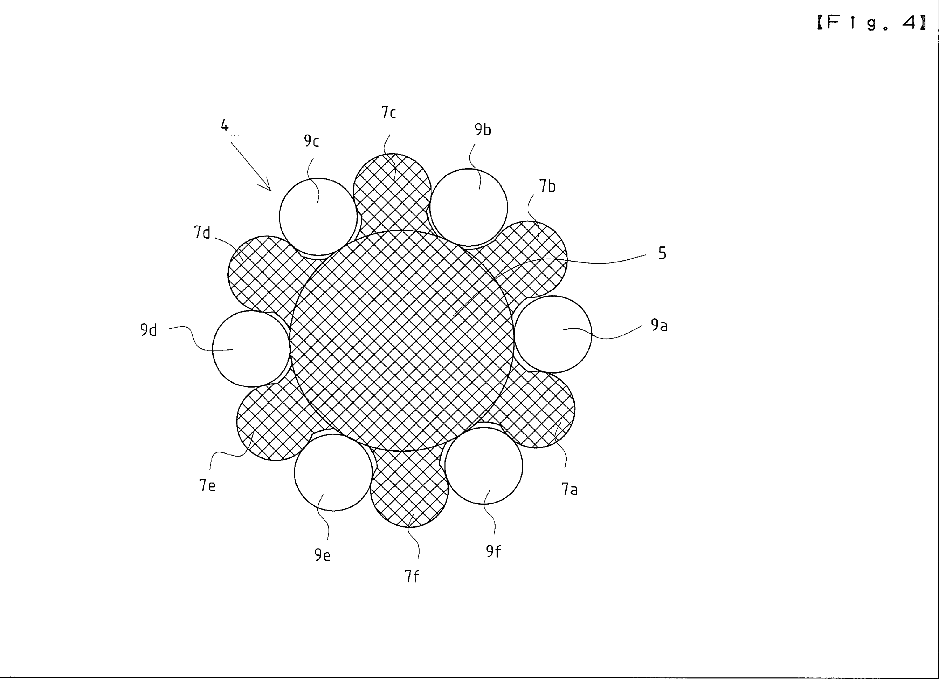

Fig. 1 shows a side view of the connection structure according to the first embodiment of the present invention, and Fig. 2 shows a cross-sectional side view of the connection structure according to the first embodiment, and Fig. 3 shows a cross-sectional view at A-A of Fig. 1, and Fig. 4 shows a cross-sectional view at B-B of Fig. 1.

Note that in order to clearly indicate that a first rod 3 and first metal element wires 9 as described below comprise the same material, and a second rod 5 and second metal element wires 7 as described below comprise the same material, only portions corresponding to the second rod 5 and the second metal wires 7 are shaded in the present embodiment.

With reference to Figs. 1 and 2, a connection structure 1 for connecting two metal rods connects the first rod 3 comprising a stainless steel alloy and the second rod 5 comprising a nickel-titanium alloy, in which a multi-thread coil 4 formed by winding the first metal element wires 9 comprising the stainless steel alloy and the second metal element wires 7 comprising the nickel-titanium alloy is arranged, the multi-thread coil 4 bestriding the first rod 3 and the second rod 5.

The multi-thread coil 4 in the present embodiment is formed by winding the first metal element wires 9 (9a, 9b, 9c, 9d, 9e, 9f) comprising 6 strands of the stainless steel alloy and the second metal element wires 7 (7a, 7b, 7c, 7d, 7e, 7f) comprising 6 strands of the nickel-titanium alloy.

Further, in the multi-thread coil 4 according to the present embodiment, the first metal element wires 9 and the second metal element wires 7 are adjacently wound one by one.

As shown in Fig. 3, the first rod 3 comprising the stainless steel alloy is welded to the first metal element wires 9 (9a, 9b, 9c, 9d, 9e, 9f) comprising the stainless steel alloy, but not welded to the second metal element wires 7 (7a, 7b, 7c, 7d, 7e, 7f) comprising the nickel-titanium alloy.

Further, with reference to Fig. 3, the first metal element wires 9 (9a, 9b, 9c, 9d, 9e, 9f) comprising the stainless steel alloy is welded to the first rod 3 so as to sandwich the second metal element wires 7 (7a, 7b, 7c, 7d, 7e, 7f) comprising the nickel-titanium alloy from both sides at a connected portion between the first rod 3 and the multi-thread coil 4.

On the other hand, the second rod 5 comprising the nickel-titanium alloy is welded to the second metal element wires 7 (7a, 7b, 7c, 7d, 7e, 7f) comprising the nickel titanium alloy, but not welded to the first metal element wires 9 (9a, 9b, 9c, 9d, 9e, 9f) comprising the stainless steel alloy as shown in Fig. 4.

Moreover, with reference to Fig. 4, the second metal element wires 7 (7a, 7b, 7c, 7d, 7e, 7f) comprising the nickel-titanium alloy is welded to the second rod 5 so as to sandwich the first metal element wires 9 (9a, 9b, 9c, 9d, 9e, 9f) comprising the stainless steel alloy from both sides at a connected portion between the second rod 5 and the multi-thread coil 4.

However, the first metal element wires 9 and the second metal element wires 7 are arranged around the cross-sectional circumferences at an equal angle so as to cover the entire cross-sectional circumferences of the first rod 3 and the second rod 5.

Further, in the connection structure 1 according to the present embodiment, the first rod 3 comprises a stainless steel alloy, and the second rod 5 comprises a nickel-titanium alloy, and the multi-thread coil 4 is formed by winding the first metal element wires 9 comprising the stainless steel alloy and the second metal element wires 7 comprising the nickel-titanium alloy. However, the configuration is not limited to this.

For example, the first rod 3 may comprise a cobalt-chromium alloy, and the second rod 5 may comprise a nickel-titanium alloy, and the multi-thread coil 4 may be formed by winding the first metal element wires 9 comprising the cobalt-chromium alloy and the second metal element wires 7 comprising the nickel-titanium alloy. Alternatively, the first rod 3 may comprise a stainless steel alloy, and the second rod 5 may comprise a cobalt-chromium alloy, and the multi-thread coil 4 may be formed by winding the first metal element wires 9 comprising the stainless steel alloy and the second metal element wires 7 comprising the cobalt-chromium alloy.

In the connection structure 1 according to the present embodiment, the multi-thread coil formed by winding the first elemental wires 9 comprising the first metal and the second element wires 7 comprising the second metal is arranged , the multi-thread coil bestriding the first rod 3 comprising the first metal such as a stainless steel alloy and the second rod 5 comprising the second metal such as a nickel-titanium alloy, and the first rod 3 is not connected to the second element wires 7 of the multi-thread coil 4, but connected to the first element wires 9 of the multi-thread coil 4, and the second rod 5 is not connected to the first element wires 9 of the multi-thread coil 4, but connected to the second element wires 7 of the multi-thread coil 4. This can improve the flexibility of a connected portion formed by the multi-thread coil.

Further, in the connection structure 1 according to the present embodiment, the multi-thread coil 4 comprises the first metal elemental wires 9 and the second metal element wires 7 adjacently wound one by one, and the first element wires are connected to the first metal so as to sandwich the second element wires, and the second element wires is connected to the second metal so as to sandwich the first element wires. This can prevent distortion of the shape of the multi-thread coil 4 as much as possible, and can improve the flexibility of the connected portion.

Moreover, the connection structure according to the present embodiment can improve the flexibility of the connected portion, and can also provide an appropriate connection even when dissimilar metals are used such as a stainless steel alloy and a nickel titanium alloy, which are difficult to be directly connected.

### Second embodiment

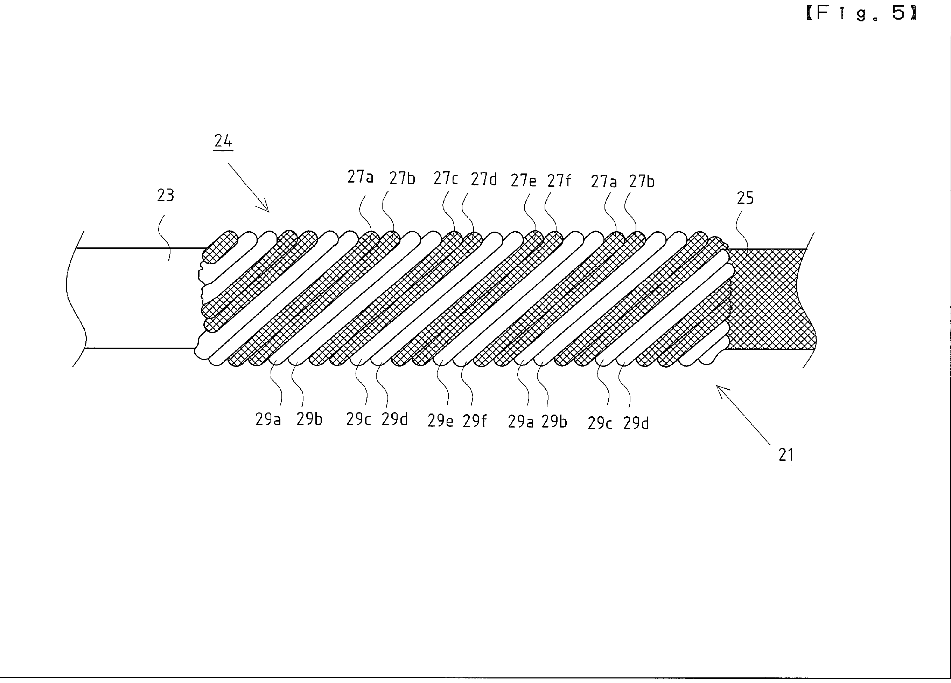

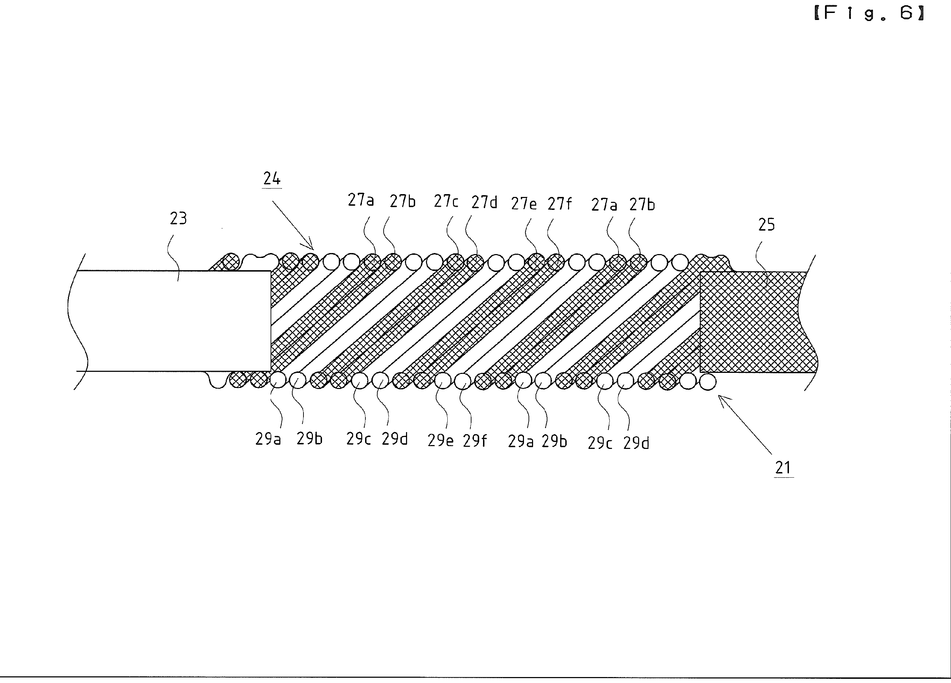

Fig. 5 shows a side view of a connection structure according to the second embodiment, and Fig. 6 shows a cross-sectional side view of the connection structure according to the second embodiment.

Note that in order to clearly indicate that a third rod 23 and a third metal element wires 29 as described below comprise the same material, and a fourth rod 25 and fourth metal element wires 27 as described below comprise the same material, only portions corresponding to the fourth rod 25 and the fourth metal wires 27 are shaded in the present embodiment.

With reference to Figs. 5 and 6, a connection structure 21 for connecting two metal rods connects the third rod 23 comprising a stainless steel alloy to the fourth rod 25 comprising a nickel-titanium alloy, in which a multi-thread coil 24 formed by winding the third metal element wires 29 comprising the stainless alloy and the fourth metal element wires 27 comprising the nickel-titanium alloy is connected , the multi-thread coil 24 bestriding the third rod 23 and the fourth rod 25.

The multi-thread coil 24 in the present embodiment is formed by winding the third metal element wires 29 (29a, 29b, 29c, 29d, 29e, 29f) comprising 6 strands of the stainless alloy and the fourth metal element wires 27 (27a, 27b, 27c, 27d, 27e, 27f) comprising 6 strands of the nickel-titanium alloy.

Further, unlike the first embodiment, the multi-thread coil 24 in the present embodiment comprises the third metal element wires 29 and the fourth metal element wires 27 adjacently wound two at a time.

In the present embodiment, the third rod 23 comprising the stainless steel alloy is welded to the third metal element wires 29 (29a, 29b, 29c, 29d, 29e, 29f) comprising the stainless steel alloy, but not welded to the fourth metal element wires 27 (27a, 27b, 27c, 27d, 27e, 27f) comprising the nickel titanium alloy as in the first embodiment.

Further, the third metal element wires 29 (29a, 29b, 29c, 29d, 29e, 29f) comprising the stainless steel alloy are welded to the third rod 23 so as to sandwich two of the fourth metal element wires 27 (27a, 27b, 27c, 27d, 27e, 27f) comprising the nickel-titanium alloy from both sides at a connected portion between the third rod 23 and the multi-thread coil 24.

On the other hand, the fourth rod 25 comprising the nickel-titanium alloy is welded to the fourth metal element wires 27 (27a, 27b, 27c, 27d, 27e, 27f) comprising the nickel titanium alloy, but not welded to the third metal element wires 29 (29a, 29b, 29c, 29d, 29e, 29f) comprising the stainless steel alloy as in the first embodiment.

Further, the fourth metal element wires 27 (27a, 27b, 27c, 27d, 27e, 27f) comprising the nickel-titanium alloy are welded to the fourth rod 25 so as to sandwich two of the third metal element wires 29 (29a, 29b, 29c, 29d, 29e, 29f) comprising the stainless steel alloy from both sides at a connected portion between the fourth rod 25 and the multi-thread coil 24.

Note that in the multi-thread coil 4 according to the present embodiment, two of the third metal element wires 29 (29a, 29b, 29c, 29d, 29e, 29f) are paired, and two of the fourth metal element wires 27 (27a, 27b, 27c, 27d, 27e, 27f) are paired, but the configuration is not limited to this. Three of the third metal element wires 29 (29a, 29b, 29c, 29d, 29e, 29f) may be bundled, and three of the fourth metal element wires 27 (27a, 27b, 27c, 27d, 27e, 27f) may be bundled.

However, the third metal element wires 29 and the fourth metal element wires 27 are arranged around the cross-sectional circumferences at an equal angle so as to cover the entire cross-sectional circumferences of the third rod 23 and the fourth rod 25.

Further, in the connection structure 21 according to the present embodiment, the third rod 23 comprises a stainless alloy, and the fourth rod 25 comprises a nickel-titanium alloy, and the multi-thread coil 24 is formed by winding the third metal element wires 29 comprising the stainless alloy and the fourth metal element wires 27 comprising the nickel-titanium alloy. However, the configuration is not limited to this.

For example, the third rod 23 may comprise a cobalt-chromium alloy, and the fourth rod 25 may comprise a nickel-titanium alloy, and the multi-thread coil 24 may be formed by winding the third metal element wires 29 comprising the cobalt-chromium alloy and the fourth metal element wires 27 comprising the nickel-titanium alloy. Alternatively, the third rod 23 may comprise a stainless steel alloy, and the fourth rod 25 may comprise a cobalt-chromium alloy, and the multi-thread coil 24 may be formed by winding the third metal element wires 29 comprising the stainless steel alloy and the fourth metal element wires 27 comprising the cobalt-chromium alloy.

In the connection structure 21 according to the present embodiment, the multi-thread coil formed by winding the third elemental wires 29 comprising the first metal and the fourth element wires 27 comprising the second metal is arranged , the multi-thread coil 24 bestriding the third rod 23 comprising the first metal such as a stainless steel alloy and the fourth rod 25 comprising the second metal such as a nickel-titanium alloy, and the third rod 23 is connected to the third element wires 29 of the multi-thread coil 24, and the fourth rod 25 is connected to the fourth element wires 27 of the multi-thread coil 24. This can improve the flexibility of a connected portion formed by the multi-thread coil 24.

Moreover, the connection structure 21 according to the present embodiment can improve the flexibility of the connected portion, and can also provide an appropriate connection even when dissimilar metals are used such as a stainless steel alloy and a nickel titanium alloy, which are difficult to be directly connected.

### Third embodiment

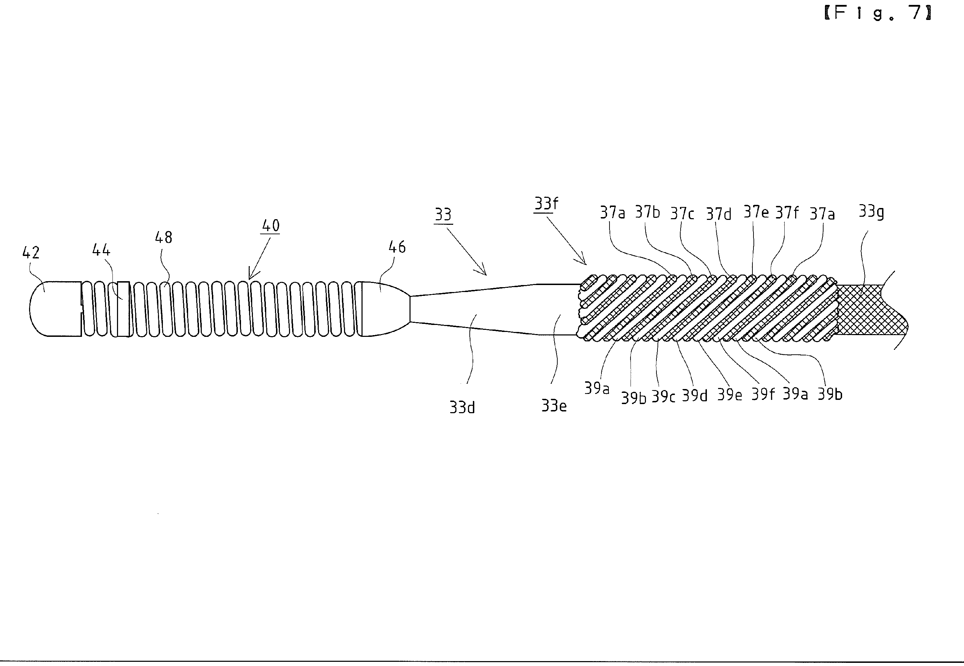

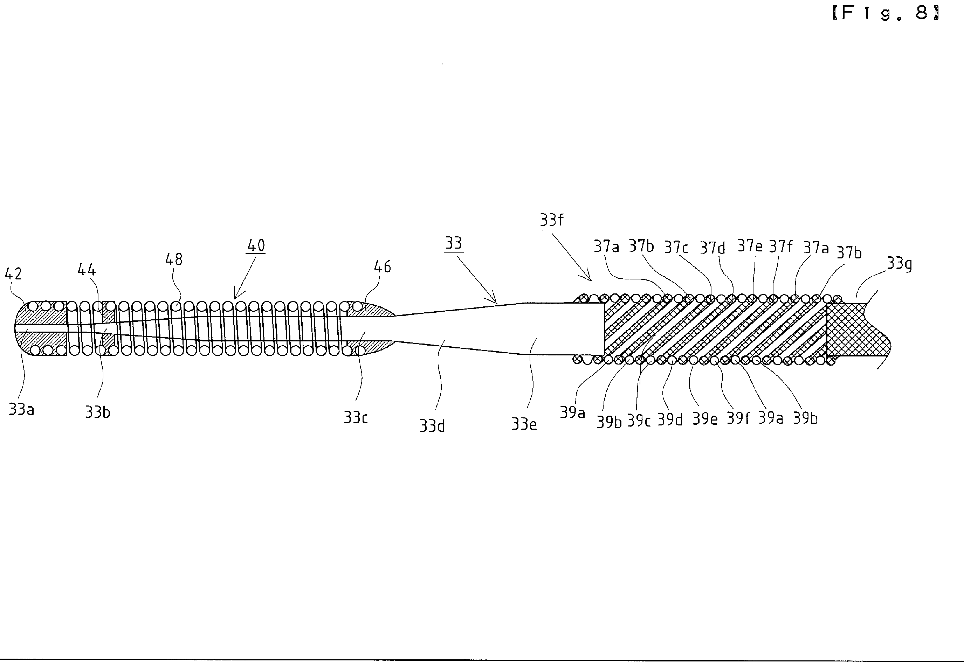

Fig. 7 shows a schematic side view of a guide wire according to the third embodiment, and Fig. 8 shows a schematic cross-sectional side view of the guide wire according to the third embodiment.

Note that in order to clearly indicate that a first cylinder portion 33a, a first tapered portion 33b, a second cylinder portion 33c, a second tapered portion 33d, a third cylinder portion 33e, and fifth metal element wires 39 of a core shaft 33 as described below comprise the same material, a fourth cylinder portion 33g and sixth metal element wires 37 of the core shaft 33 as described below comprise the same material, only portions corresponding the fourth cylinder portion 33g and the sixth metal element wires 37 of the core shaft 33 are shaded in the present embodiment.

With reference to Figs. 7 and 8, a guide wire 40 comprises the core shaft 33 and a coil body 48 covering the front end of the core shaft 33.

The coil body 48 is a single-thread coil body comprising a stainless steel alloy.

The core shaft 33 comprises, as listed from the front end, the first cylinder portion 33a, the first tapered portion 33b, the second cylinder portion 33c, the second tapered portion 33d, the third cylinder portion 33e and the fourth cylinder portion 33g; and a multi-thread coil body 33f arranged bestriding the third cylinder portion 33e and the fourth cylinder portion 33g.

Here, the first cylinder portion 33a, the first tapered portion 33b, the second cylinder portion 33c, the second tapered portion 33d, and the third cylinder portion 33e are each an elongated metal rod body with a round cross-section comprising a stainless steel alloy, and the fourth cylinder portion 33g is an elongated metal rod body with a round cross-section comprising a nickel-titanium alloy.

Further, the multi-thread coil 33f is formed by winding the fifth metal element wires 39 (39a, 39b, 39c, 39d, 39e, 39f) comprising 6 strands of the stainless steel alloy and the sixth metal element wires 37 (37a, 37b, 37c, 37d, 37e, 37f) comprising 6 strands of the nickel-titanium alloy.

The coil body 48 is a single-thread coil body comprising the stainless steel alloy, in which the front end part thereof is brazed to the front end of the first cylinder portion 33a of the core shaft 33 to form a front brazing portion 42.

Further, the base end part of the coil body 48 is brazed to the second cylinder portion 33c of the core shaft 33 to form a base end brazing portion 46, and the middle part of the coil body 48 is brazed to the first tapered portion 33b of the core shaft 33 to form a middle brazing portion 44.

The multi-thread coil 33f according to the present embodiment is formed by winding the fifth metal element wires 39 (39a, 39b, 39c, 39d, 39e, 39f) comprising 6 strands of the stainless alloy and the sixth metal element wires 37 (37a, 37b, 37c, 37d, 37e, 37f) comprising 6 strands of the nickel-titanium alloy.

Further, in the multi-thread coil 33f according to the present embodiment, the fifth metal element wires 39 and the sixth metal element wires 37 are adjacently wound one by one.

In the present embodiment, the third cylinder portion 33e comprising the stainless steel alloy is welded to the fifth metal element wires 39 (39a, 39b, 39c, 39d, 39e, and 39f) comprising the stainless steel alloy of the multi-thread coil 33f, but not welded to the sixth metal element wires 37 (37a, 37b, 37c, 37d, 37e, 37f) comprising the nickel-titanium alloy of the multi-thread coil 33f.

Further, the fifth metal element wires 39 (39a, 39b, 39c, 39d, 39e, 39f) are welded to the third cylinder portion 33e so as to sandwich the sixth metal element wires 37 (37a, 37b, 37c, 37d, 37e, 37f) from both sides at a connected portion of the third cylinder portion 33e and the multi-thread coil 33f.

On the other hand, the fourth cylinder portion 33g comprising the nickel-titanium alloy is welded to the sixth metal element wires 37 (37a, 37b, 37c, 37d, 37e, 37f) comprising the nickel-titanium alloy of the multi-thread coil 33f, but not welded to the fifth metal element wires 39 (39a, 39b, 39c, 39d, 39e, 39f) comprising the stainless steel alloy of the multi-thread coil 33f.

Further, the sixth metal element wires 37 (37a, 37b, 37c, 37d, 37e, 37f) are welded to the fourth cylinder portion 33g so as to sandwich the fifth metal element wires 39 (39a, 39b, 39c, 39d, 39e, 39f) from both sides at a connected portion of the fourth cylinder portion 33g and the multi-thread coil 33f.

However, the fifth metal element wires 39 and the sixth metal element wires 37 are arranged around the cross-sectional circumferences in an equal angle so as to cover the entire cross-sectional circumferences of the third cylinder portion 33e and the fourth cylinder portion 33g of the core shaft 33.

Further, in the present embodiment, the third cylinder portion 33e of the core shaft 33 comprises a stainless steel alloy, and the fourth cylinder portion 33g of the core shaft 33 comprises a nickel titanium alloy, and the multi-thread coil 33f is formed by winding the fifth metal element wires 39 comprising the stainless steel alloy and the sixth metal element wires 37 comprising the nickel titanium alloy. However, the configuration is not limited to this.

For example, the third cylinder portion 33e of the core shaft 33 may comprise a cobalt-chromium alloy, and the fourth cylinder portion of the core shaft 33 may comprise a nickel-titanium alloy, and the multi-thread coil 33f may be formed by winding the fifth metal element wires 39 comprising the cobalt-chromium alloy and the sixth metal element wires 37 comprising the nickel-titanium alloy. Alternatively, the third cylinder portion 33e of the core shaft 33 may comprise a stainless steel alloy, and the fourth cylinder portion of the core shaft 33 may comprise a cobalt-chromium alloy, and the multi-thread coil 33f may be formed by winding the fifth metal element wires 39 comprising the stainless steel alloy and the sixth metal element wires 37 comprising the cobalt-chromium alloy.

In the guide wire 40 according to the present embodiment, the multi-thread coil 33f formed by winding the fifth metal element wires 39 comprising the first metal and the sixth metal element wires 37 comprising the second metal is arranged, the multi-thread coil 33f bestriding the third cylinder portion 33e of the core shaft 33 comprising the first metal such as a stainless steel alloy and the fourth cylinder portion 33g comprising the second metal such as a nickel titanium alloy, and the third cylinder portion 33e of the core shaft 33 is connected to the fifth metal element wires 39 of the multi-thread coil 33f, and the fourth cylinder portion 33g is connected to the sixth metal element wires 37 of the multi-thread coil 33f. This can improve the flexibility of a connected portion formed by the multi-thread coil 33f, enabling an appropriate followability along a winding blood vessel.

Further, in the guide wire 40 according to the present embodiment, the multi-thread coil 33f comprises the fifth metal element wires 39 and the sixth metal element wires 37 adjacently wound one by one, and the fifth metal element wires 39 are connected to the third cylinder portion 33e of the core shaft 33 so as to sandwich the sixth metal element wires 37, and the sixth metal element wires 37 are connected to the fourth cylinder portion 33g so as to sandwich the fifth metal element wires 39. This can prevent distortion of the shape of the multi-thread coil 33f as much as possible, and can also improve the flexibility of the connected portion.

Note that in the present embodiment, an example is presented where the connection structure according to the first embodiment is used in a guide wire, but the configuration is not limited to this. The connection structure according to the second embodiment may also be used in a guide wire. In that case, the advantageous effects of the connection structure according to the second embodiment will be manifested therein.

The metal element wires of the multi-thread coil are welded to the rod bodies or the core shaft in the embodiments described above, but they may be connected by a method other than welding. However, welding is preferred considering that metals can easily be connected.

### DESCRIPTION OF SYMBOLS

1, 21 Connection structure
3, 5, 23, 25 Rod body
4, 24, 33f Multi-thread coil
7, 9, 27, 29, 37, 39 Metal element wire
33 Core shaft
40 Guide wire
48 Coil body

## Claims

1. A connection structure (1, 21) between a first metal rod (3, 23, 33a-33e) comprising a first metal and a second metal rod (5, 25, 33g) comprising a second metal, wherein
a multi-thread coil (4, 24, 33f) formed by winding first element wires (9, 29, 39) comprising the first metal, and second element wires (7, 27, 37) comprising the second metal is arranged, the multi-thread coil bestriding the first metal rod (3, 23, 33a-33e) and the second metal rod (5, 35, 33g), and
the first metal rod (3, 23, 33a-33e) is connected to the first element wires (9, 29, 39) of the multi-thread coil (4, 24, 33f), and the second metal rod (5, 35, 33g) is connected to the second element wires (7, 27, 37) of the multi-thread coil (4, 24, 33f), wherein
the first element wires (9, 29, 39) and the second element wires (7, 27, 37) are arranged around cross-sectional circumferences at an equal angle so as to cover the entire cross-sectional circumferences of the first metal rod (3, 23, 33a-33e) and the second metal rod (5, 25, 33g).

2. The connection structure (1) according to claim 1, wherein the multi-thread coil (4, 33f) comprises the first element wires (9, 39) and the second element wires (7, 37) adjacently wound one by one, and
the first element wires (9, 39) are connected to the first metal rod (3, 33a-33e) so as to sandwich the second element wires (7, 37), and the second element wires (7, 37) are connected to the second metal rod (5, 33g) so as to sandwich the first element wires (9, 39).

3. The connection structure (1, 21) according to claim 1 or 2, wherein the first metal comprises a stainless steel alloy, and the second metal comprises a nickel-titanium alloy.

4. A guide wire (40), comprising a core shaft (33) having the connection structure (1, 21) according to any one of claims 1 to 3, and a coil body (48) covering a front end of the core shaft (33) and connected to the first metal rod (33a-33e) or the second metal rod (33g).

## Patentansprüche

1. Verbindungsstruktur (1, 21) zwischen einem ersten Metallstab (3, 23, 33a-33e), der ein erstes Metall aufweist, und einem zweiten Metallstab (5, 25, 33g), der ein zweites Metall aufweist, wobei
eine mehrgängige Spule (4, 24, 33f) angeordnet ist, die durch eine Wicklung aus ersten Elementdrähten (9, 29, 39), die das erste Metall aufweisen, und zweiten Elementdrähten (7, 27, 37), die das zweite Metall aufweisen, gebildet ist, wobei die mehrgängige Spule den ersten Metallstab (3, 23, 33a-33e) und den zweiten Metallstab (5, 35, 33g) umringt, und
der erste Metallstab (3, 23, 33a-33e) mit den ersten Elementdrähten (9, 29, 39) der mehrgängigen Spule (4, 24, 33f) und der zweite Metallstab (5, 35, 33g) mit den zweiten Elementdrähten (7, 27, 37) der mehrgängigen Spule (4, 24, 33f) verbunden ist, wobei
die ersten Elementdrähte (9, 29, 39) und die zweiten Elementdrähte (7, 27, 37) in einem gleichen Winkel um Querschnittsumfänge herum derart angeordnet sind, dass sie die gesamten Querschnittsumfänge des ersten Metallstabs (3, 23, 33a-33e) und des zweiten Metallstabs (5, 25, 33g) erfassen.

2. Verbindungsstruktur (1) nach Anspruch 1, wobei die mehrgängige Spule (4, 33f) die ersten Elementdrähte (9, 39) und die zweiten Elementdrähte (7, 37) umfasst, die nebeneinander gewickelt sind, und
die ersten Elementdrähte (9, 39) mit dem ersten Metallstab (3, 33a-33e) derart verbunden sind, dass sie die zweiten Elementdrähte (7, 37) sandwichartig einschließen, und die zweiten Elementdrähte (7, 37) mit dem zweiten Metallstab (5, 33g) derart verbunden sind, dass sie die ersten Elementdrähte (9, 39) sandwichartig einschließen.

3. Verbindungsstruktur (1, 21) nach Anspruch 1 oder 2, wobei das erste Metall eine Edelstahllegierung und das zweite Metall eine Nickel-Titan-Legierung enthält.

4. Führungsdraht (40), umfassend einen Kernschaft (33), der eine Verbindungsstruktur (1, 21) nach einem der Ansprüche 1 bis 3 und einen Spulenkörper (48) aufweist, der ein vorderes Ende der Kernwelle (33) erfasst und mit dem ersten Metallstab (33a-33e) oder dem zweiten Metallstab (33g) verbunden ist.

## Revendications

1. Structure de liaison (1, 21) entre une première tige de métal (3, 23, 33a-33e) comprenant un premier métal et une seconde tige de métal (5, 25, 33g) comprenant un second métal, dans laquelle
une bobine à fils multiples (4, 24, 33f) formée par enroulement de premiers fils d'élément (9, 29, 39) comprenant le premier métal, et de seconds fils d'élément (7, 27, 37) comprenant le second métal est agencée, la bobine à fils multiples enjambant la première tige de métal (3, 23, 33a-33e) et la seconde tige de métal (5, 35, 33g), et
la première tige de métal (3, 23, 33a-33e) est reliée aux premiers fils d'élément (9, 29, 39) de la bobine à fils multiples (4, 24, 33f), et la seconde tige de métal (5, 35, 33g) est reliée aux seconds fils d'élément (7, 27, 37) de la bobine à fils multiples (4, 24, 33f), dans laquelle
les premiers fils d'élément (9, 29, 39) et les seconds fils d'élément (7, 27, 37) sont agencés autour de circonférences de section transversale à un angle égal de sorte à couvrir les circonférences de section transversale entières de la première tige de métal (3, 23, 33a-33e) et de la seconde tige de métal (5, 25, 33g).

2. Structure de liaison (1) selon la revendication 1, dans laquelle la bobine à fils multiples (4, 33f) comprend les premiers fils d'élément (9, 39) et les seconds éléments de fils (7, 37) enroulés de manière adjacente un par un, et
les premiers fils d'élément (9, 39) sont reliés à la première tige de métal (3, 33a-33e) de sorte à prendre en sandwich les seconds fils d'élément (7, 37), et les seconds fils d'élément (7, 37) sont reliés à la seconde tige de métal (5, 33g) de sorte à prendre en sandwich les premiers fils d'élément (9, 39).

3. Structure de liaison (1, 21) selon la revendication 1 ou 2, dans laquelle le premier métal comprend un alliage d'acier inoxydable, et le second métal comprend un alliage de nickel-titane.

4. Fil-guide (40) comprenant une tige centrale (33) présentant la structure de liaison (1, 21) selon l'une quelconque des revendications 1 à 3, et un corps de bobine (48) couvrant une extrémité avant de la tige centrale (33) et relié à la première tige de métal (33a, 33e) ou à la seconde tige de métal (33g).
